(19) Europäisches Patentamt
European Patent Office
Office européen des brevets

(11) Publication number: **0 010 893**
**B1**

(12) # EUROPEAN PATENT SPECIFICATION

(45) Date of publication of patent specification: **08.02.84**

(51) Int. Cl.[3]: **C 07 D 277/48, A 61 K 31/425**

(21) Application number: **79302211.2**

(22) Date of filing: **15.10.79**

(54) **Antisecretory branched-chain heterocyclic guanidine derivatives, processes for their manufacture, intermediates, and pharmaceutical compositions containing said derivatives.**

(30) Priority: **16.10.78 GB 4070578**

(43) Date of publication of application:
**14.05.80 Bulletin 80/10**

(45) Publication of the grant of the patent:
**08.02.84 Bulletin 84/6**

(84) Designated Contracting States:
**AT BE CH DE FR GB IT NL SE**

(56) References cited:
FR - A - 2 276 042
FR - A - 2 311 536
GB - A - 1 533 380
US - A - 3 975 530
US - A - 4 000 302
US - A - 4 070 475
US - A - 4 104 382

Burger's Medicinal Chemistry, Part III, 4th edition, pp. 487-551

(73) Proprietor: **IMPERIAL CHEMICAL INDUSTRIES PLC**
**Imperial Chemical House Millbank**
**London SW1P 3JF (GB)**

(73) Proprietor: **ICI AMERICAS INC**
**Concord Pike & New Murphy Road**
**Wilmington Delaware 19897 (US)**

(72) Inventor: **Yellin, Tobias Oregon**
**4 Waterford Way**
**Wallingford, PA 19086 (US)**
Inventor: **Gilman, David John**
**4 Gloucester Close**
**Tytherington Macclesfield, Cheshire (GB)**
Inventor: **Jones, Derrick Fleet**
**22 Badger Road**
**Tytherington Macclesfield, Cheshire (GB)**

(74) Representative: **Brown, Ivor James Stewart et al,**
**Imperial Chemical Industries PLC Legal Department: Patents PO Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Antisecretory branched-chain heterocyclic guanidine derivatives, processes for their manufacture, intermediates, and pharmaceutical compositions containing said derivatives

This invention relates to heterocyclic derivatives which are histamine H-2 antagonists and which inhibit gastric acid secretion.

It is postulated that the physiologically-active compound histamine, which occurs naturally within the animal body, is able to combine, in the course of exerting its activity, with certain specific receptors of which there are at least two distinct and separate types. The first has been named the H-1 receptor (Ash and Schild, *Brit. J. Pharmac.,* 1966, *27,* 427) and the action of histamine at this receptor is blocked (antagonised) by classical "antihistamine" drugs such as mepyramine. The second histamine receptor has been named the H-2 receptor (Black *et al., Nature,* 1972, *236,* 385) and the action of histamine at this receptor is blocked by drugs such as cimetidine. It is known that one of the results of the blockade of the action of histamine at the H-2 receptor is the inhibition of the secretion of gastric acid and a compound which possesses this ability is therefore useful in the treatment of peptic ulcers and other conditions caused or exacerbated by gastric acidity.

In UK Patent 1,341,375 there are described histamine H-2 receptor antagonists which are heterocyclic derivatives having a branched side chain to the end of which is attached, for example, a urea or guanidine residue. It has now been discovered that if a guanidino radical is substituted in the 2-position of the hetercyclic ring carrying such a side chain in the 4-position there are produced compounds which are potent histamine H-2 receptor antagonists.

According to the invention there is provided a heterocyclic derivative of the formula:-

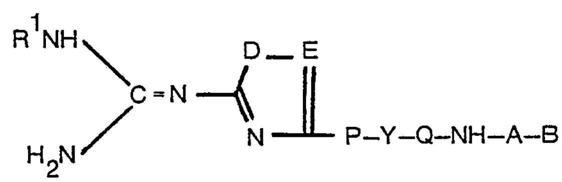

I

in which
D is an oxygen or sulphur atom or an NH radical;
E is a CH radical or, when D is an oxygen or sulphur atom, E is a nitrogen atom;
Y is a sulphur or oxygen atom, a direct bond or a methylene or sulphinyl radical;
P is a direct bond or an unbranched alkylene chain of 1 to 4 carbon atoms optionally substituted by 1 or 2 alkyl radicals of 1 to 4 carbon atoms, and Q is an unbranched alkylene chain of 1 to 4 carbon atoms optionally substituted by 1 or 2 alkyl radicals of 1 to 4 carbon atoms, provided at least one of P and Q is substituted by at least one alkyl radical, that the number of alkyl radical substituents on P and Q together is a maximum of 2, that when Y is a sulphur or oxygen atom or a sulphinyl radical P is an optionally substituted alkylene chain, and that when Y is an oxygen atom or a sulphinyl radical Q is an optionally substituted alkylene chain of 2 to 4 carbon atoms; $R^1$ is a hydrogen atom or an alkyl radical of 1 to 10 carbon atoms;
A is a 3,4-dioxocyclobuten-1,2-diyl radical or a radical of the formula $C=Z$ in which Z is a sulphur or oxygen atom or a radical of the formula NCN, $NNO_2$, $CHNO_2$, $NCONH_2$, $C(CN)_2$, $NCOR^2$, $NCO_2R^2$, $NSO_2R^2$ or $NR^3$ in which $R^2$ is an alkyl radical of 1 to 6 carbon atoms or an aryl radical of 6 to 12 carbon atoms and $R^3$ is a hydrogen atom or an alkyl radical of 1 to 6 carbon atoms;
B is an alkoxy or alkylthio radical of 1 to 6 carbon atoms or a radical of the formula $NR^4R^5$ in which $R^4$ and $R^5$, which may be the same or different, are hydrogen atoms, alkyl radicals of 1 to 10 carbon atoms, alkenyl or alkynyl radicals of 3 to 6 carbon atoms in which the double or triple bond respectively is separated from the nitrogen atom of $NR^4R^5$ by at least one carbon atom, (primary hydroxy)alkyl or (primary amino)alkyl radicals of 2 to 6 carbon atoms or cycloalkyl radicals of 3 to 6 carbon atoms, or $R^4$ and $R^5$ are joined to form, together with the nitrogen atom to which they are attached, a 5- or 6-membered saturated ring which optionally contains an additional oxygen or NH radical;
and the pharmaceutically-acceptable acid-addition salts thereof.

It is to be understood that in the above formula I and throughout this specification, although the double bonds in both side chains have been inserted in particular positions, various other tautomeric forms are possible, and this invention includes such tautomeric forms within its scope, both in terms of the compound of the invention and in terms of the manufacturing processes.

When P or Q is an alkylene chain substituted by one alkyl radical, or by two non-identical alkyl radicals on the same carbon atom, the compound of the formula I exists in two enantiomeric forms. When P and/or Q is substituted by two alkyl radicals on different carbon atoms, the compound of the formula I exists in two diastereoisomeric forms each of which exists in two enantiomeric forms. When Y is a sulphinyl radical the number of enantiomeric forms in the compound of the formula I is doubled. The biological activity, as hereinafter defined, of these enantiomeric forms may differ and it is therefore to be understood that this invention encompasses both the racemate of the formula I, including any possible diastereoisomeric forms, and any enantiomeric form which possesses the disclosed biological

activity, it being a matter of common general knowledge to one skilled in the art how to separate diastereoisomeric forms and how to separate a racemate into its enantiomers and determined the biological activity of each.

A particular value for $R^1$ is a hydrogen atom or a methyl or n-butyl radical.

A particular value for the optional alkyl radical substituent on P or Q is a methyl radical.

A particular value for $R^2$ is a methyl, phenyl or *p*-tolyl radical.

A particular value for $R^3$ is a hydrogen atom or a methyl radical.

A particular value for B is a methoxy, ethoxy or methylthio radical or a radical of the formula $NR^4R^5$ in which $R^4$ and $R^5$, which may be the same or different, are hydrogen atoms or methyl, ethyl, n-propyl, i-propyl, n-hexyl, allyl, propargyl, 2-hydroxyethyl, 3-hydroxypropyl, 2-aminoethyl, cyclopropyl, cyclobutyl or cyclohexyl radicals or $R^4$ and $R^5$ are joined to form a pyrrolidine, piperidine, piperazine or morpholine ring.

The following are 6 preferred features of the heterocyclic derivative of the formula I. When any of these 6 features is taken, either singly or in combination, with the other general or particular features of the heterocyclic derivatives of the formula I listed above, there are obtained preferred sub-groups of compounds within the above general definition.

1. Y is a sulphur atom and P is an optionally-substituted methylene radical and Q is an optionally substituted ethylene radical.

2. Y is a direct bond, P is an optionally substituted methylene radical and Q is an optionally-substituted propylene or butylene radical.

3. The number of alkyl radical substitutents on P and Q together is 1.

4. $R^1$ is a hydrogen atom and B is a radical of the formula $NR^4R^5$ in which $R^5$ is a hydrogen atom and $R^4$ is a hydrogen atom or a methyl, ethyl, n-propyl, i-propyl, n-hexyl, allyl, propargyl, 2-hydroxyethyl, 3-hydroxypropyl, 2-aminoethyl, cyclopropyl, cyclobutyl or cyclohexyl radical.

5. D is a sulphur atom and E is a CH radical or a nitrogen atom.

6. A is a radical of the formula $C=Z$ in which Z is a radical of the formula NCN.

Particular compounds of the invention are described in the Examples and of these preferred compounds are 2-guanidino-4-[5-(2-cyano-3-methylguanidino)-2-methylpentyl]thiazole, 2-guanidino-4-[2-(2-cyano-3-methylguanidino)-1-methylethylthiomethyl]-thiazole and 2-guanidino-4-[2-(2-cyano-3-methylguanidino)-2-methylethylthiomethyl]thiazole, and the pharmaceutically-acceptable acid-addition salts thereof.

A suitable pharmaceutically-acceptable acid-addition salt of the heterocyclic derivative of the invention is, for example, a salt formed with hydrochloric, hydrobromic, phosphoric, sulphuric, acetic, citric or maleic acid.

The heterocyclic derivative of the invention may be manufactured by methods known in themselves for the manufacture of chemically analogous compounds. The following processes, A, B, D, E, P, Q, Y, $R^1$, $R^2$, $R^3$, $R^4$ and $R^5$ having the meanings stated above unless indicated otherwise, are therefore provided as further features of the invention.

The process of the invention is characterised by:-

(a) reaction of a compound of the formula II:-

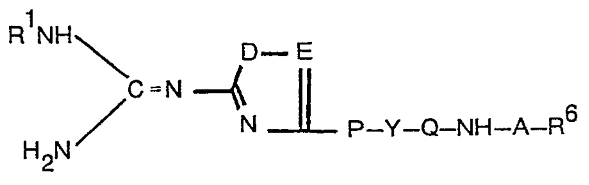

II

in which $R^6$ is a displaceable radical with a compound of the formula B—H;

(b) for those compounds in which A is a radical of the formula $C=Z$ in which Z is a sulphur or oxygen atom and B is a radical of the formula $NR^4R^5$ in which $R^5$ is a hydrogen atom and $R^4$ has the value stated above other than a hydroxyalkyl or aminoalkyl radical, reaction of a compound of the formula III:-

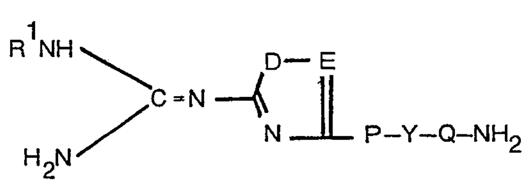

III

with a compound of the formula $R^7—N=C=G$ in which G is an oxygen or sulphur atom and $R^7$ is a hydrogen atom or an alkyl, alkenyl, alkynyl or cycloalkyl radical;

(c) reaction of a compound of the formula III with a compound of the formula IV:-

$R^6$—A—B IV

in which $R^6$ is a displaceable radical;

(d) for those compounds in which A is a radical of the formula C = Z in which Z is a radical of the formula $NCONH_2$ and B is a radical of the formula $NR^4R^5$, hydrolysis of a compound of the formula I in which A is a radical of the formul C = Z in which Z is a radical of the formula NCN and B is a radical of the formula $NR^4R^5$;

(e) for those compounds in which Y is a sulphinyl radical, oxidation of a compound of the formula I in which Y is a sulphur atom; or

(f) for a compound which is an optically-active enantiomer, resolution of the racemic compound of the formula I or use of any of processes (a) to (d) inclusive in which the starting material is itself a resolved isomer:

whereafter when the compound of the formula I is obtained in the form of the free base and a salt is required, the free base is reacted with an acid which affords a pharmaceutically-acceptable anion.

Process (a) may be carried out using an excess of B—H, that is using an excess of the amine $R^4R^5NH$, optionally in the presence of a diluent or solvent such as water, methanol, ethanol or pyridine, or using an excess of the compound $R^8$—OH or $R^8$—SH in which $R^8$ is an alkyl radical of 1 to 6 carbon atoms, preferably in the form of a salt such as the sodium salt in the same alcohol or thiol as diluent or solvent. $R^6$ may, for example, be an alkoxy or alkylthio radical of 1 to 6 carbon atoms, for example a methoxy, ethoxy or methylthio radical. The process may be accelerated or completed by the application of heat, for example by heating to the boiling point of the diluent or solvent.

Process (b) may be carried out using an excess of the isocyanate or isothiocyanate $R^7 - N = C = G$. When G is a sulphur atom, the reaction is preferably carried out in a diluent or solvent such as methanol or ethanol. When G is an oxygen atom, a non-alcoholic diluent or solvent must be used. The process may be accelerated or completed by the application of heat, for example by heating to the boiling point of the diluent or solvent.

Process (c) may be carried out using an excess of the compound of the formula IV in a diluent or solvent such as methanol, ethanol or acetonitrile. $R^6$ may, for example, be an alkoxy or alkylthio radical of 1 to 6 carbon atoms, for example a methoxy, ethoxy or methylthio radical. The reaction may be accelerated or completed by the application of heat, for example by heating to the boiling point of the diluent or solvent.

Process (d) may be carried out using a dilute mineral acid, for example dilute hydrochloric acid, in a diluent or solvent such as water. The reaction may be accelerated or completed by the application of heat, for example by heating to the boiling point of the diluent or solvent.

Process (e) may be carried out using a mild oxidising agent such as sodium metaperiodate in a diluent or solvent such as aqueous methanol or aqueous ethanol.

The starting material of the formula II for use in process (a) may be obtained by reaction of a compound of the formula III with a compound of the formula:-

$R^6$—A—$R^6$ V

in which $R^6$ is a displaceable radical, for example an alkoxy or alkylthio radical, for example as set out in Example 3, 4 or 5.

When D is a sulphur atom or NH radical, E is a CH radical and Y is a direct bond or a methylene radical, the starting material of the formula III for use in process (b) or (c) may be prepared by reaction of a bromoketone of the formula:-

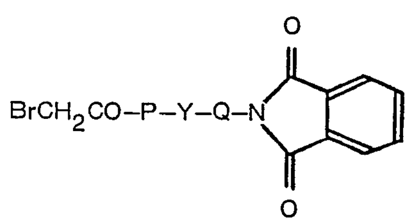

VI

with a compound of the formula:-

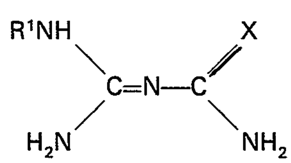

VII

in which X is a sulphur atom or an NH radical, followed by hydrolysis of the phthalimido radical, for example as set out in Example 1 or 2.

When D is a sulphur or NH radical, E is a CH radical and Y is a sulphur or oxygen atom, the starting material of the formula III for use in process (b) or (c) may be prepared by reaction of a compound of the formula:-

$$ClCH_2CO—P—Cl \qquad VIII$$

with a compound of the formula VII followed by reaction of the product, the compound of the formula:-

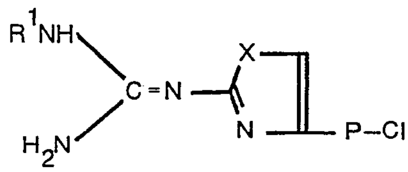

IX

with a compound of the formula:-

$$HG—Q—NH_2 \qquad X$$

in which G is an oxygen or sulphur atom, for example as set out in Example 4 or 5.

When D is an oxygen atom, E is a CH radical and Y is a direct bond or a methylene radical, the starting material of the formula III may be prepared by reaction of a compound of the formula

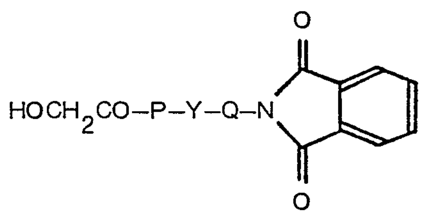

XI

with a compound of the formula:-

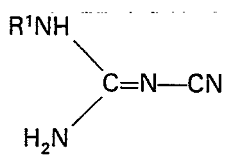

(XII)

followed by hydrolysis of the phthalimido radical.

When D is an oxygen atom, E is a CH radical and Y is a sulphur or oxygen atom, the starting material of the formula III may be prepared by reaction of a compound of the formula:-

$$HOCH_2CO—P—Cl \qquad XIII$$

with a compound of the formula XII followed by reaction of the product, the compound of the formula:-

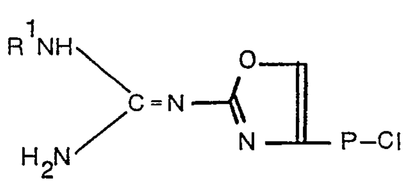

XIV

with a compound of the formula X.

When D is an oxygen atom, E is a nitrogen atom and Y is a direct bond or a methylene radical, the starting material of the formula III may be prepared by reaction of a compound of the formula:-

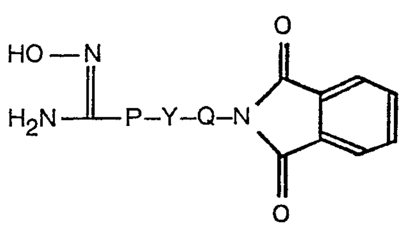

XV

with a compound of the formula XII followed by hydrolysis of the phthalimido residue in the product.

When D is an oxygen atom, E is a nitrogen atom and Y is an oxygen or sulphur atom, the starting material of the formula III may be prepared by reaction of a compound of the formula:-

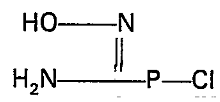

XVI

with a compound of the formula XII followed by reaction of the product, the compound of the formula:-

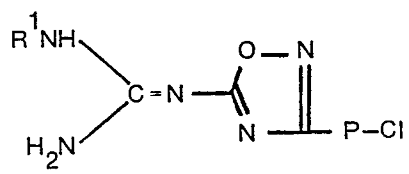

XVII

with a compound of the formula X.

When D is a sulphur atom, E is a nitrogen atom and Y is a direct bond or a methylene radical, the starting material of the formula III may be prepared by reaction of a compound of the formula:-

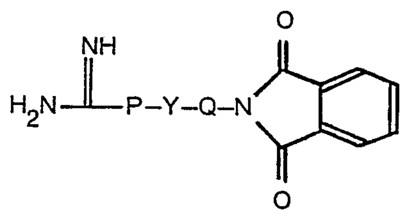

XVIII

with trichloromethane sulphenyl chloride, followed by reaction of the product, the compound of the formula:-

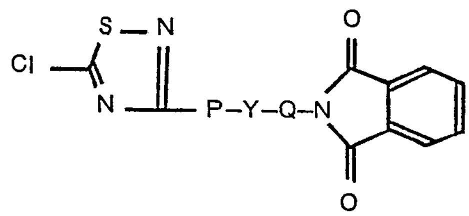

XIX

with a compound of the formula:-

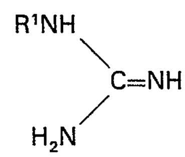

XX

followed by hydrolysis of the phthalimido radical.

When D is a sulphur atom, E is a nitrogen atom and Y is an oxygen or sulphur atom, the starting material of the formula III may be prepared by reaction of a compound of the formula:-

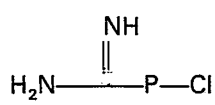

XXI

with trichloromethanesulphenyl chloride followed by reaction of the product, the compound of the formula:-

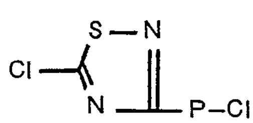

XXII

with a compound of the formula XX. The product from this reaction is then reacted with a compound of the formula X.

When Y is a sulphinyl radical, the starting material of the formula III may be prepared by oxidation of the corresponding compound in which Y is a sulphur atom.

Intermediates of the formula III and acid-addition salts thereof are also within the scope of this invention.

As noted above, the heterocyclic derivative of the invention is a histamine H-2 antagoinst, inhibits the secretion of gastric acid in warm-blooded animals and is therefor useful in the treatment of peptic ulcers and other conditions caused or exacerbated by gastric acidity, including stress ulcers and gastrointestinal bleeding due to trauma.

The histamine H-2 antagonist activity may be demonstrated on standard tests, for example by the ability of the compound of the formula I to inhibit the histamine-induced positive chronotropic response in the spontaneously beating right atrium of the guinea pig or by its ability to inhibit the histamine-induced increase in the level of cyclic AMP (in the presence of a phosphodiesterase inhibitor) in a free cell suspension obtained from canine gastric mucosa.

The guinea pig atrium test is carried out as follows:-

A guinea pig right atrium is suspended at 1 g. tension (isometric) in a thermostatically-controlled (30°C.) tissue bath (25 ml.) containing oxygenated (95% $O_2$; 5% $CO_2$) Krebs-Hanseleit buffer (pH 7.4). The tissue is allowed to stabilise over 1 hour during which time it is washed 2—4 times. Individual contractions are recorded with a force-displacement transducer through a strain gauge coupler, and instantaneous rates are monitored with a cardiotachometer. A control response to 1 $\mu$M histamine is obtained after which the tissue is washed 3 times and allowed to re-equilibrate to basal rate. After reequilibration for 15 minutes, the test compound is added to the desired final concentration. Ten minutes after addition of the compound histamine (1 $\mu$M) is again added and the response to histamine in the presence of antagonist is compared to the histamine control response. The result is expressed as a percentage of the histamine control response. Thereafter the apparent dissociation constant of the H-2 antagonist is determined by standard procedures.

All the compounds exemplified in this specification are active on the guinea pig atrium test at or below a bath concentration of 10 $\mu$M.

The inhibition of the secretion of gastric acid may be demonstrated in standard tests, for example by the ability of the compound of the formula I, when doses intravenously, intragastrically or orally, to inhibit the secretion of acidic gastric juice in, for example, rats, cats or dogs provided with gastric fistulae and whose gastric secretion is stimulated by the administration of a secretagogue, for example pentagastrin or histamine.

The test in dogs is carried out as follows:-

A female pure bred beagle (9—12 kg.) having a chronic gastric fistula is fasted overnight with water *ad lib.* During the experiment the dog is lightly restrained in a standing position. When studying the test compound by the intravenous route, the fistula is opened and, after ascertaining the absence of basal secretion over a period of 30 minutes, a continuous intravenous infusion of secretagogue (0.5 $\mu$mole/kg/hour of histamine or 2$\mu$g./kg./hour pentagastrin) in saline (15 ml./hour) is begun. Gastric acid samples are collected every 15 minutes. The volume of each sample is measured and a 1 ml. aliquot is titrated to neutrality with 0.1 N NaOH to determine acid concentration. When a plateau of secretion is reached, (1—2 hours) the test compound is administered intravenously in saline and gastric acid samples are collected for a further 2—3 hours during which time the infusion of the secretagogue continues uninterrupted.

When studying the test compound by the intragastric route, the absence of basal secretion over a period of 30 minutes is ascertained and the test compound, contained in 25 ml. of 0.5% w/v hydroxypropyl methylcellulose and 0.1% w/v 'Tween' 80 in water ('Tween' is a Trade Mark), is instilled into the stomach through a fistula dosing plug. One hour later, the fistula is reopened and intravenous infusion of a secretagogue, as described above, is immediately begun. Gastric acid samples are measured as described above and the approach of acid secretion to a plateau is compared to that of a control animal which is dosed intragastrically only with the dosing vehicle.

When studying the test compound by the oral route, it is administered in a gelatin capsule washed down with 15 ml. of water. One hour later, the fistula is opened and intravenous infusion of the secretagogue is immediately begun. Gastric acid samples are measured as above and the approach of acid secretion to a plateau is compared to that of an undosed control animal.

The results obtained in the atrium test are predictive of activity in the dog test.

No overt toxicity or side effects were noted during the dog tests.

According to a further feature of the invention there is provided a pharmaceutical composition which comprises a heterocyclic derivative of the invention in association with a non-toxic pharmaceutically-acceptable diluent or carrier.

The pharmaceutical composition may, for example, be in a form suitable for oral, rectal, parenteral or topical administration, for which purposes it may be formulated by means known to the art into the form of, for example, tablets, capsules, aqueous or oily solutions or suspensions, emulsions, dispersible

powders, suppositories, sterile injectable aqueous or oily solutions or suspensions, gels, creams, ointments or lotions.

In addition to the heterocyclic derivative of the formula I, the pharmaceutical composition of the invention for oral, rectal or parenteral administration may also contain, or be co-administered with, one or more known drugs selected from antacids, for example aluminium hydroxide — mangesium hydroxide mixtures; antipepsin compounds, for example pepstatin; other histamine H-2 antagonists, for example cimetidine; ulcer healing agents, for example carbenoxolone or bismuth salts; anti-inflammatory agents, for example ibuprofen, indomethacin, naproxen or aspirin (Trade Mark); prostaglandins, for example 16,16-dimethylprostaglandin $E_2$; classical antihistamines (histamine H-1 antagonists), for example mepyramine or diphenhydramine; anti-cholinergic agents, for example atropine or propantheline bromide; and anxiolytic agents, for example diazepam, chlordiazepoxide or phenobarbital.

The pharmaceutical composition of the invention for topical administration may also contain, in addition to the heterocyclic derivative, one or more classical antihistamines (histamine H-1 antagonists), for example mepyramine or diphenhydramine and/or one or more steroidal anti-inflammatory agents, for example fluocinolone or triamcinolone.

A topical formulation may contain 1—10% w/w of the heterocyclic derivative of the invention. A preferred pharmaceutical composition of the invention is one suitable for oral administration in unit dosage form, for example a tablet or capsule which contains between 10 mg. and 500 mg. of the heterocyclic derivative, or one suitable for intravenous, subcutaneous or intramuscular injection, for example a sterile injectable containing between 0.1% and 10% w/w of the heterocyclic derivative.

The pharmaceutical composition of the invention will normally be administered to man for the treatment of peptic ulcers and other conditions caused or exacerbated by gastric acidity in the same general manner as that employed for cimetidine, due allowance being made in terms of dose levels for the potency of the heterocyclic derivative of the present invention relative to cimetidine. Thus each patient will receive an oral dose of between 20 mg. and 1500 mg. and preferably between 50 mg. and 500 mg. of heterocyclic derivative or an intravenous, subcutaneous or intramuscular dose of between 2.5 mg, and 150 mg, and preferably between 10 mg. and 50 mg. of the heterocyclic derivative, the composition being administered 2 to 4 times per day. The rectal dose will be approximately the same as the oral dose. The composition may be administered less frequently when it contains an amount of heterocyclic derivative which is a multiple of the amount which is effective when given 2—4 times per day.

The invention is illustrated, but not limited by the following Examples in which the temperatures are in degrees Centigrade:-

Example 1

A solution of 2-guanidino-4-(4-amino-1-methylbutyl)thiazole dihydrobromide (1.14 g.) in methanol (10 ml.) was treated with a solution of potassium hydroxide (0.378 g.) in methanol (10 ml.). The solution was evaporated to dryness and the residue extracted with warm ethanol (50 ml.). The filtered ethanol solution was concentrated to 5 ml. and treated with methylisothiocyanate (0.24 g.). After stirring at room temperature for 16 hours the reaction mixture was evaporated and the residue subjected to preparative thin layer chromatography on plates of silica gel GF (Analtech. Inc. Newark, Delaware U.S.A.). The chromatogram was developed with chloroform/methanol/ammonia (s.g. 0.88) 80:20:0.5 v/v/v and the appropriate region of the chromatogram was isolated and extracted with hot ethanol/chloroform 3:1 v/v. Evaporation of the filtered ethanol extract and recrystallisation of the residue from ethanol-petroleum ether (b.p. 30—60°) gave 0.18 g. of 2-guanidino-4-[4-(3-methylthioureido)-1-methylbutyl]thiazole, m.p. 145—148° (decomp.).

The 2-guanidino-4-(4-amino-1-methylbutyl)-thiazole dihydrobromide used as starting material may be prepared as follows:-

2-Methyl-5-phthalimidopentanoic acid [S. Sugasawa and M. Murayama, *Chem. Pharm. Bull., 6,* 194-200, (1958)] (1.5 g) was treated with thionyl chloride (1.4 g.) and the mixture stirred at room temperature for 1.5 hours. The residual acid chloride, obtained on removal of excess thionyl chloride by evaporation under reduced pressure, was dissolved in dry ether (25 ml.) and this solution was added to a dry ethereal solution of diazomethane (prepared from 21.4 g. of DIAZALD Aldrich Chemical Company Inc. Milwarkee, Wisconsin according to the procedure described in "Reagents for Organic Synthesis Vol 1, Fieser and Fieser, pp. 191—192) and the mixture allowed to stand at room temperature for 16 hours. The oily diazoketone which was obtained on evaporation of the reaction mixture was dissolved in acetone (10 ml.) and to this solution was added hydrobromic acid (48% w/v) dropwise until no further effervescence occurred. After the addition of water (10 ml.) the reaction mixture was extracted with ether (3 x 25 ml). and the combined ethereal extracts were dried over magnesium sulphate and evaporated to give N-(6-bromo-4-methyl-5-oxohexyl)phthalimide as a yellow oil. This oil (1.9 g.) in hot ethanol (10 ml.) was treated with a hot solution of amidinothiourea (0.65 g.) in ethanol (30 ml.) and the resulting solution was heated under reflux for 0.75 hour. Concentration of the reaction mixture to low volume and cooling gave 1.3 g of 2-guanidino-4-(1-methyl-4-phthalimidobutyl)thiazole hydrobromide, m.p. 185—187°. This material, suspended in methanol (10 ml.), was treated with 2.5 N sodium

hydroxide (25 ml.) and the mixture heated under reflux for 0.25 hour. The reaction mixture was adjusted to pH 1 by the addition of hydrobromic acid (48% w/v) and reflux continued for 0.5 hour. The pH of the refluxing mixture was then readjusted to pH 12 (with 2.5 N sodium hydroxide) for 0.25 hours and returned to pH 1 with hydrobromic acid for 0.5 hour. After removal of most of the methanol by evaporation under reduced pressure, the acidic aqueous residue was diluted with water (15 ml.) and extracted with ethyl acetate (4 x 35 ml.). The aqueous solution was evaporated to dryness and the solid residue was extracted with hot ethanol (3 x 50 ml.). The combined ethanol extracts were filtered and evaporated to give 1.14 g. of 2-guanidino-4-(4-amino-1-methylbutyl)-thiazole dihydrobromide as a light brown solid.

Example 2

A solution of 2-guanidino-4-(5-amino-2-methylpentyl)thaizole hydrobromide hydrochloride (0.6 g.) in methanol (10 ml.) was treated with a solution of potassium hydroxide (0.25 g.) in methanol (10 ml.). The solution was evaporated and the residue extracted with warm ethanol (40 ml.). The filtered ethanol solution was concentrated to 5 ml., treated with methylisothiocyanate (0.21 g.) and stirred at room temperature for 16 hours. The residue obtained on evaporation of the reaction mixture was subjected to preparative thin layer chromatography on plates of silica gel GF (Analtech Inc. Newark, Delaware, U.S.A.). The chromatogram was developed with chloroform/methanol/ammonia (s.g. 0.88) 135:15:0.5, v/v/v and the appropriate region of the chromatogram was isolated and extracted with a warm mixture of ethanol/chloroform (3:1 v/v). The residue obtained on removal of the solvents was dissolved in ethanol (3 ml.) and treated with a solution of maleic acid (0.22 g).) (approx. one mole. equivalent) in ethanol (3 ml.). The resulting solution was diluted with ether to give 0.34 g. of 2-guanidino-4-[5-(3-methylthioureido)-2-methyl-pentyl]thiazole hydrogen maleate, m.p. 142—145°.

The 2-guanidino-4-(5-amino-2-methylpentyl)-thiazole hydrobromide hydrochloride used as starting material may be prepared as follows:-

2-Methyl-5-phthalimidopentanoic acid [S. Sugasawa and M. Murayama, *Chem. Pharm. Bull, 6,* 194—200, (1958)] (4.5 g.) was treated with thionyl chloride (4.1 g.) and the mixture stirred at room temperature for 2.5 hours. The residual acid chloride, obtained on removal of excess thionyl chloride, was dissolved in dry ether (50 ml.) and this solution added to a dry ethereal solution of diazomethane (prepared from 21.4 g. of DIAZALD — Aldrich Chemical Company Inc. Milwaukee, Wisconsin — according to the procedure described in "Reagents for Organic Synthesis" Vol 1, Fieser and Fieser, pp. 191—192) and triethylamine (1.74 g.) maintained at —60°. After 0.5 hour the reaction mixture was allowed to attain room temperature and allowed to stand for 16 hours. The filtered solution was evaporated to give the diazoketone as a yellow oil (4.9 g). This oil was treated with collidine (25 ml.) and benzyl alcohol (25 ml.) and the mixture placed in an oil bath maintained at 185°. The mixture was stirred until effervescence ceased (approx. 0.25 hour), cooled and diluted with ether (150 ml.). The ethereal solution was extracted with 2N hydrochloric acid (3 x 50 ml.), washed with aqueous sodium bicarbonate and dried over magnesium sulphate. Evaporation of the solvent gave a dark red oil. This was subjected to short path distillation (Kugelrohr apparatus) at 90°/25 mm Hg to remove benzyl alcohol and further distillation gave benzyl 3-methyl-6-phthalimidohexanoate as a yellow oil (5.8 g.), pot. temp. 190—200° at 0.15 mm Hg. This oil in ethanol (100 ml.) was hydrogenated over 10% w/w palladium on charcoal at room temperature and 50 p.s.i. until hydrogen uptake ceased. The filtered reaction mixture was evaporated and the residue crystallised from ethylacetate/petroleum ether (b.p. 30—60°) to give 3-methyl-6-phthalimidohexanoic acid, m.p. 98—100°. This acid (4.0 g.) was converted to the diazoketone derivative by procedures analogous to those applied to 2-methyl-5-phthalimido pentanoic acid in Example 1. The diazoketone which was obtained as an orange oil (4.3 g.) was dissolved in acetone (15 ml.) and treated dropwise with hydrobromic acid (48% w/v) until evolution of nitrogen ceased. Dilution of the reaction mixture with water precipitated 4.7 g. of N-(7-bromo-4-methyl-6-oxoheptyl)phthalimide, m.p. 99—103°. This bromoketone (2.3 g.), dissolved in hot ethanol (30 ml.), was treated with amidinothiourea (0.77 g.) in hot ethanol (50 ml.) and the resulting solution heated under reflux for 0.5 hour. Concentration of the reaction mixture followed by dilution with ether gave 2-guanidino-4-[2-methyl-5-phthalimidopentyl]-thiazole hydrobromide (2.3 g.), m.p. 160—164°. This material, suspended in methanol (25 ml.), was treated with 2.5 N sodium hydroxide (6 ml.) and the mixture heated under reflux for 0.25 hour. The reaction mixture was adjusted to pH 1 by the addition of conc. hydrochloric acid and reflux continued for 0.5 hour. The pH of the refluxing mixture was then readjusted to pH 12 (with 2.5 N sodium hydroxide) for 0.25 hour and returned to pH 2 (with conc. hydrochloric acid) for 0.5 hour. After removal of methanol by evaporation under reduced pressure the reaction mixture was diluted with water (20 ml.) and extracted with ethyl acetate (3 x 25 ml.). The aqueous solution was evaporated to dryness and the solid residue extracted with hot ethanol (3 x 50 ml.). The combined ethanol extracts were filtered and evaporated to give 1.2 g. of 2-guanidino-4-[5-amino-2-methylpentyl]thiazole hydrobromide hydrochloride as light brown glass.

Example 3

A solution of 2-guanidino-4-(5-amino-2-methylpentyl)thiazole hydrobromide hydrochloride (0.6 g.) in methanol (10 ml.) was treated with a solution of potassium hydroxide (0.25 g.) in methanol

(10 ml.). The solution was evaporated and the residue extracted with warm ethanol (50 ml.). The filtered ethanolic solution was concentrated to approx. 5 ml. and treated with dimethyl (cyanoimido)dithiocarbonate (0.3 g.) and the mixture stirred at room temperature for 16 hours. The 2-guanidino-4-[5-(3-cyano-2-methylisothioureido)-2-methylpentyl]thiazole formed as an intermediate was not isolated. A solution of methylamine in ethanol (33% w/v, 10 ml.) was added and the reaction mixture allowed to stand for 17 hours. The residue, obtained on evaportion of the solvent, was subjected to preparative thin layer chromatography on plates of silica gel GF (Analtech Inc., Newark, Delaware). The chromatogram was developed with chloroform/methanol/ammonia (s.g. 0.88) 127:23:0.5 v/v/v and the appropriate region of the chromatogram isolated and extracted with ethanol/chloroform (3:1 v/v). The residue obtained on evaporation of the filtered extract was dissolved in ethanol (2 ml.) and treated with a solution of maleic acid (0.2 g.) in ethanol 2 ml. The resulting solution was diluted with ether to give 0.4 g. of 2-guanidino-4-[5-(2-cyano-3-methylguanidino)-2-methylpentyl]thiazole hydrogen maleate, m.p. 131—134°.

Example 4

A solution of 1-aminopropane-2-thiol hydrochloride (4.8 g.) in ethanol (25 ml.) was added dropwise to a stirred, ice-cooled solution of sodium (1.75 g.) in ethanol (40 ml.) under a nitrogen atmosphere. The mixture was stirred at 0° for 1,5 hours, and a solution of 2-guanidino-4-chloromethylthiazole hydrochloride (4.25 g,) in ethanol (40 ml.) was added dropwise, keeping the temperature at 0—2°. After the addition the mixture was stirred at room temperature for 18 hours, filtered, and the solvent removed by evaporation under reduced pressure. The residue was dissolved in methanol (30 ml.), and the stirred solution was treated successively with triethylamine (1.3 g.) and then dimethyl (cyanoimido)dithiocarbonate (1.8 g.). After stirring at room temperature for 2 days, 33% w/v methanolic methylamine (10 ml.) was added and stirring continued for 18 hours. The mixture was evaporated under reduced pressure to give an oil, which was dissolved in acetone (10 ml.) and treated with a solution of maleic acid (1.4 g.) in acetone (10 ml.) to give a white precipitate which was filtered off and recrystallised from water to give 2-guanidino-4-[2-(2-cyano-3-methylguanidino)-1-methylethylthiomethyl]thiazole hydrogen maleate, m.p. 168—169°.

Example 5

Crude 2-guanidino-4-(2-amino-2-methylethylthiomethyl)thiazole hydrochloride (0.83 g.) in methanol (10 ml.) and triethylamine (0.5 ml.) was treated with dimethyl (cyanoimido)dithiocarbonate (0.36 g.) and the solution was stirred overnight at room temperature. To the resulting solution containing 2-guanidino-4-[2-(2-cyano-3-methylisothioureido)-2-methylethylthiomethyl]thiazole was added a solution of methylamine in ethanol (33% w/v, 10 ml.) and this solution was stirred at room temperature for three days. The residue left after evaporation of the solvent was subjected to preparative thin layer chromatography (Merck 60F—254 plates developed with ethyl acetate/ethanol/ammonia (s.g. 0.88) 6:1:1 v/v/v). A band of $R_f$ about 0.5 was collected and extracted with ethanol. Evaporation of the filtered extract gave an oil which was dissolved in acetone (5 ml.) and treated with a solution of maleic acid (0.1 g.) in acetone (10 ml.) to give a precipitate which was filtered off and recrystallised from ethanol to give 2-guanidino-4-[2-(2-cyano-3-methylguanidino)-2-methylethylthiomethyl]thiazole hydrogen maleate as a white solid, m.p. 130—133°.

The crude 2-guanidino-4-(2-amino-2-methylethylthiomethyl)thiazole hydrochloride used as starting material may be prepared as follows:-

1-Amino-2-hydroxypropane hydrobromide (7.8 g; prepared by dissolving 1-amino-2-hydroxypropane in excess concentrated hydrobromic acid, and evaporating the solution to dryness) was treated with phosphorus tribromide (6.8 g.) and the mixture was heated until the reaction was complete, as indicated by the presence of only one phase in the mixture as opposed to the original two phases. Excess phosphorus tribromide was removed by gentle heating under water pump pressure. The residue was cooled, washed with ehter, and dissolved in ethanol. The solution was filtered and allowed to evaporate at room temperature, giving a clear oil which solidified to a white solid on standing in a vacuum desiccator.

This crude 1-amino-2-bromopropane hydrobromide (11.3 g.) was dissolved in water (20 ml.) and treated with 3.6 N aqueous sodium hydroxide (29 ml.). The solution was stirred in an ice bath, carbon disulphide (4.3 g.) was added dropwise, and the mixture stirred for a further 1.5 hours. The supernatant solution was decanted from the sticky precipitate which formed and the residue washed with water (10 ml.). Recrystallisation from water gave a mixture of 2-mercapto-4-methylthiazoline and 2-mercapto-5-methylthiazoline as white needles, m.p. 93—94°.

A mixture of the above 4- and 5-methylthiazolines (1.3 g.), concentrated hydrochloric acid (15 ml.) and water (7 ml.) was heated under reflux for 7 days. The mixture was cooled and evaporated to dryness to give a mixture containing 2-amino-1-mercaptopropane hydrochloride as an oil, which was dissolved in dry ethanol (10 ml.) and added to a stirred, ice-cooled solution of sodium (0.5 g.) in ethanol (10 ml.). The resulting suspension was treated dropwise with a solution of 4-chloromethyl-2-guanidinothiazole hydrochloride (1.1 g.) in ethanol (20 ml.), and then stirred overnight while warming to room temperature. The solution was filtered, the filtrate acidified with hydrogen chloride in isopropanol and the solution evaporated to dryness to give a mixture containing 2-guanidino-4-(2-amino-2-methylethylthiomethyl)thiazole hydrochloride as a pale yellow oil which crystallised on trituration with a little ethanol.

**Claims for the Contracting States: BE CH DE FR GB IT NL SE**

1. A heterocyclic derivative of the formula:-

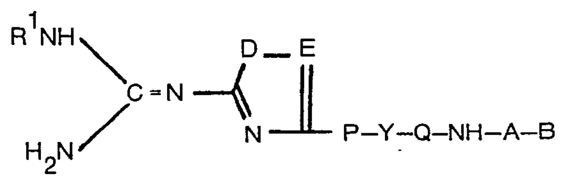

I

in which
D is an oxygen or sulphur atom or an NH radical;
E is a CH radical or, when D is an oxygen or sulphur atom, E is a nitrogen atom;
Y is a sulphur or oxygen atom, a direct bond or a methylene or sulphinyl radical;
P is a direct bond, or an unbranched alkylene chain of 1 to 4 carbon atoms optionally substituted by 1 or 2 alkyl radicals of 1 to 4 carbon atoms, and Q is an unbranched alkylene chain of 1 to 4 carbon atoms optionally substituted by 1 or 2 alkyl radicals of 1 to 4 carbon atoms, provided at least one of P and Q is substituted by at least one alkyl radical, that the number of alkyl radical substituents on P and Q together is a maximum of 2, that when Y is a sulphur or oxygen atom or a sulphinyl radical P is an optionally substituted alkylene chain, and that when Y is an oxygen atom or a sulphinyl radical Q is an optionally substituted alkylene chain of 2 to 4 carbon atoms;
$R^1$ is a hydrogen atom or an alkyl radical of 1 to 10 carbon atoms;
A is a 3,4-dioxocyclobuten-1,2-diyl radical or a radical of the formula C=Z in which Z is a sulphur or oxygen atom or a radical of the formula NCN, $NNO_2$, $CHNO_2$, $NCONH_2$, $C(CN)_2$, $NCOR^2$, $NCO_2R^2$, $NSO_2R^2$ or $NR^3$ in which $R^2$ is an alkyl radical of 1 to 6 carbon atoms or an aryl radical of 6 to 12 carbon atoms and $R^3$ is a hydrogen atom or an alkyl radical of 1 to 6 carbon atoms;
B is an alkoxy or alkylthio radical of 1 to 6 carbon atoms or a radical of the formula $NR^4R^5$ in which $R^4$ and $R^5$, which may be the same or different, are hydrogen atoms, alkyl radicals of 1 to 10 carbon atoms, alkenyl or alkynyl radicals of 3 to 6 carbon atoms in which the double or triple bond respectively is separated from the nitrogen atom of $NR^4R^5$ by at least one carbon atom, (primary hydroxy)alkyl or (primary amino)alkyl radicals of 2 to 6 carbon atoms or cycloalkyl radicals of 3 to 6 carbon atoms, or $R^4$ and $R^5$ are joined to form, together with the nitrogen atom to which they are attached, a 5- or 6 membered saturated ring which optionally contains an additional oxygen atom or NH radical: and the pharmaceutically-acceptable acid-addition salts thereof.

2. A heterocyclic derivative as claimed in claim 1 in which $R^1$ is a hydrogen atom or a methyl or n-butyl radical; P is optionally substituted by 1 or 2 methyl radicals; Q is optionally substituted by 1 or 2 methyl radicals; $R^2$ is a methyl, phenyl or p-tolyl radical; $R^3$ is a hydrogen atom or a methyl radical; B is a methoxy, ethoxy or methylthio radical or a radical of the formula $NR^4R^5$ in which $R^4$ and $R^5$, which may be the same or different, are hydrogen atoms or methyl, ethyl, n-propyl, i-propyl, n-hexyl, allyl, propargyl, 2-hydroxyethyl, 3-hydroxypropyl, 2-aminoethyl, cyclopropyl, cyclobutyl or cyclohexyl radicals, or $R^4$ and $R^5$ are joined to form, together with the nitrogen atom to which they are attached a pyrrolidine, piperidine, piperazine or morpholine ring.

3. A heterocyclic derivative as claimed in claim 1 or 2 in which Y is a sulphur atom, P is an optionally substituted methylene radical and Q is an optionally substituted ethylene radical.

4. A heterocyclic derivative as claimed in claim 1 or 2 in which Y is a direct bond, P is an optionally substituted methylene radical and Q is an optionally substituted propylene or butylene radical.

5. A heterocyclic derivative as claimed in claim 3 or 4 in which the number of alkyl radical substituents on P and Q together is 1.

6. A heterocyclic derivative as claimed in any of claims 1 to 5 in which $R^1$ is a hydrogen atom and B is a radical of the formula $NR^4R^5$ in which $R^5$ is a hydrogen atom and $R^4$ is a hydrogen atom or a methyl, ethyl, n-propyl, i-propyl, n-hexyl, allyl, propargyl, 2-hydroxyethyl, 3-hydroxypropyl, 2-aminoethyl, cyclopropyl, cyclobutyl or cyclohexyl radical.

7. A heterocyclic derivative as claimed in any of claims 1 to 6 in which D is a sulphur atom and E is a CH radical or a nitrogen atom.

8. 2-guanidino-4-[5-(2-cyano-3-methylguanidino)-2-methylpentyl]thiazole, 2-guanidino-4-[2-(2-cyano-3-methylguanidino)-1-methyl-ethylthiomethyl]thiazole and 2-guanidino-4-[2-(2-cyano-3-methylguanidino)-2-methylethylthiomethyl]thiazole, and the pharmaceutically-acceptable acid addition salts thereof.

9. A process for the manufacture of a heterocyclic derivative as claimed in claim 1 characterised by:-
(a) reaction of a compound of the formula II:-

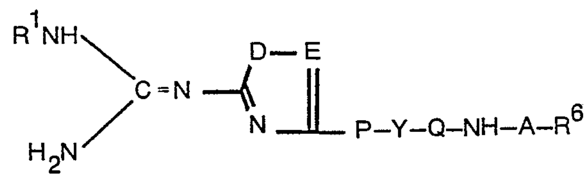

II

in which $R^6$ is a displaceable radical with a compound of the formula B—H;
(b) for those compounds in which A is a radical of the formula C=Z in which Z is a sulphur or oxygen atom and B is a radical of the formula $NR^4R^5$ in which $R^5$ is a hydrogen atom and $R^4$ has the value stated in claim 1 other than a hydroxyalkyl or aminoalkyl radical, reaction of a compound of the formula III:-

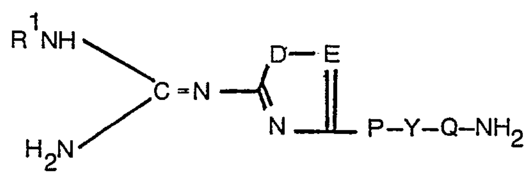

III

with a compound of the formula $R^7$—N=C=G in which G is an oxygen or sulphur atom and $R^7$ is a hydrogen atom or an alkyl, alkenyl, alkynyl or cycloalkyl radical;
(c) reaction of a compound of the formula III with a compound of the formula IV:-

$$R^6—A—B \qquad IV$$

in which $R^6$ is a displaceable radical;
(d) for those compounds in which A is a radical of the formula C=Z in which Z is a radical of the formula $NCONH_2$ and B is a radical of the formula $NR^4R^5$, hydrolysis of a compound of the formula I in which A is a radical of the formula C=Z in which Z is a radical of the formula NCN and B is a radical of the formula $NR^4R^5$;
(e) for those compounds in which Y is a sulphinyl radical, oxidation of a compound of the formula I in which Y is a sulphur atom; or
(f) for a compound which is an optically-active enantiomer, resolution of the racemic compound of the formula I or use of any of processes (a) to (d) inclusive in which the starting material is itself a resolved isomer:

whereafter when the compound of the formula I is obtained in the form of the free base and a salt is required, the free base is reacted with an acid which affords a pharmaceutically-acceptable anion.

10. A pharmaceutical composition comprising a heterocyclic derivative as claimed in claim 1 in association with a non-toxic pharmaceutically-acceptable diluent or carrier.

11. A heterocyclic derivative of the formula:-

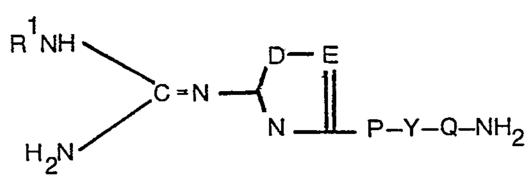

III

wherein
D is an oxygen or sulphur atom or an NH radical;
E is a CH radical or, when D is an oxygen or sulphur atom, E is a nitrogen atom;
Y is a sulphur or oxygen atom, a direct bond or a methylene or sulphinyl radical;
P is a direct bond, or an unbranched alkylene chain of 1 to 4 carbon atoms optionally substituted by 1 or 2 alkyl radicals of 1 to 4 carbon atoms, and Q is an unbranched alkylene chain of 1 to 4 carbon atoms optionally substituted by 1 or 2 alkyl radicals of 1 to 4 carbon atoms, provided at least one of P and Q is substituted by at least one alkyl radical, that the number of alkyl radical substituents on P and Q together is a maximum of 2, that when Y is a sulphur or oxygen atom or a sulphinyl radical P is an optionally substituted alkylene chain, and that when Y is an oxygen atom or a sulphinyl radical Q is an optionally substituted alkylene chain of 2 to 4 carbon atoms; and $R^1$ is a hydrogen atom or an alkyl radical of 1 to 10 carbon atoms;
and the acid-addition salts thereof.

**Claim for the Contracting State: AT**

A process for the manufacture of a heterocyclic derivative of the formula:-

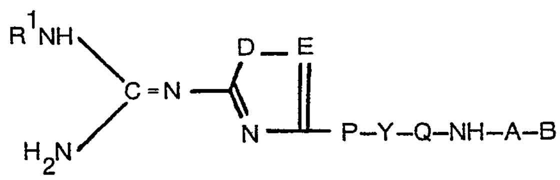

I

in which
D is an oxygen or sulphur atom or an NH radical;
E is a CH radical or, when D is an oxygen or sulphur atom, E is a nitrogen atom;
Y is a sulphur or oxygen atom, a direct bond or a methylene or sulphinyl radical;
P is a direct bond, or an unbranched alkylene chain of 1 to 4 carbon atoms optionally substituted by 1 or 2 alkyl radicals of 1 to 4 carbon atoms, and Q is an unbranched alkylene chain of 1 to 4 carbon atoms optionally substituted by 1 or 2 alkyl radicals of 1 to 4 carbon atoms, provided at least one of P and Q is substituted by at least one alkyl radical, that the number of alkyl radical substituents on P and Q together is a maximum of 2, that when Y is a sulphur or oxygen atom or a sulphinyl radical P is an optionally substituted alkylene chain, and that when Y is an oxygen atom or a sulphinyl radical Q is an optionally substituted alkylene chain of 2 to 4 carbon atoms;
$R^1$ is a hydrogen atom or an alkyl radical of 1 to 10 carbon atoms;
A is a 3-4-dioxocyclobuten-1,2-diyl radical or a radical of the formula C=Z in which Z is a sulphur or oxygen atom or a radical of the formula NCN, $NNO_2$, $CHNO_2$, $NCONH_2$, $C(CN)_2$, $NCOR^2$, $NCO_2R^2$, $NSO_2R^2$ or $NR^3$ in which $R^2$ is an alkyl radical of 1 to 6 carbon atoms or an aryl radical of 6 to 12 carbon atoms and $R^3$ is a hydrogen atom or an alkyl radical of 1 to 6 carbon atoms;
B is an alkoxy or alkylthio radical of 1 to 6 carbon atoms or a radical of the formula $NR^4R^5$ in which $R^4$ and $R^5$, which may be the same or different, are hydrogen atoms, alkyl radicals of 1 to 10 carbon atoms, alkenyl or alkynyl radicals of 3 to 6 carbon atoms in which the double or triple bond respectively is separated from the nitrogen atom of $NR^4R^5$ by at least one carbon atom, (primary hydroxy)alkyl or (primary amino)alkyl radicals of 2 to 6 carbon atoms or cycloalkyl radicals of 3 to 6 carbon atoms, or $R^4$ and $R^5$ are joined to form, together with the nitrogen atom to which they are attached, a 5- or 6-membered saturated ring which optionally contains an additional oxygen atom or NH radical; and the pharmaceutically-acceptable acid-addition salts thereof;
characterised by:-
(a) reaction of a compound of the formula:-

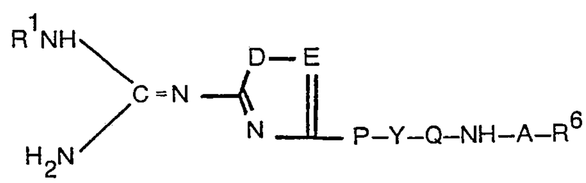

II

in which $R^6$ is a displaceable radical with a compound of the formula B—H;
(b) for those compounds in which A is a radical of the formula C=Z in which Z is a sulphur or oxygen atom and B is a radical of the formula $NR^4R^5$ in which $R^5$ is a hydrogen atom and $R^4$ has the value stated above other than a hydroxyalkyl or aminoalkyl radical, reaction of a compound of the formula:-

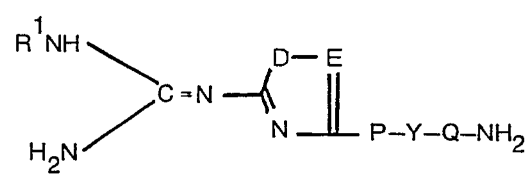

III

with a compound of the formula $R^7$—N=C=G in which G is an oxygen or sulphur atom and $R^7$ is a hydrogen atom or an alkyl, alkenyl, alkynyl or cycloalkyl radical;
(c) reaction of a compound of the formula III with a compound of the formula:-

$$R^6—A—B \qquad IV$$

in which $R^6$ is a displaceable radical;

(d) for those compounds in which A is a radical of the formula C=Z in which Z is a radical of the formula $NCONH_2$ and B is a radical of the formula $NR^4R^5$, hydrolysis of a compound of the formula I in which A is a radical of the formula C=Z in which Z is a radical of the formula NCN and B is a radical of the formula $NR^4R^5$;

(e) for those compounds in which Y is a sulphinyl radical, oxidation of a compound of the formula I in which Y is a sulphur atom; or

(f) for a compound which is an optically-active enantiomer, resolution of the racemic compound of the formula I or use of any of processes (a) to (d) inclusive in which the starting material is itself a resolved isomer:

whereafter when the compound of the formula I is obtained in the form of the free base and a salt is required, the free base is reacted with an acid which affords a pharmaceutically-acceptable anion.

**Patentansprüche für die Vertragsstaaten: BE CH DE FR GB IT NL SE**

1. Heterocyclisches Derivat der Formel:

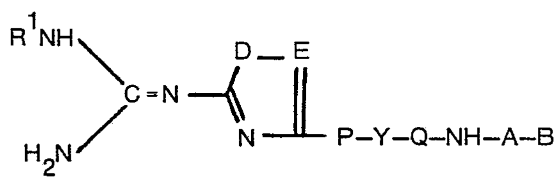

I

worin

D für ein Sauerstoff- oder Schwefelatom oder ein NH -Radikal steht;

E für ein CH -Radikal oder, wenn D ein Sauerstoff- oder Schwefelatom bedeutet, ein Stickstoffatom steht;

Y für ein Schwefel- oder Sauerstoffatom, eine direkte Bindung oder ein Methylen- oder Sulfinylradikal steht;

P für eine direkte Bindung oder eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, und Q für eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, mit der Maßgabe, daß mindestens eines der Symbole P und Q durch mindestens ein Alkylradikal substituiert ist, daß die Anzahl der Alkylradikalsubstituenten an P und Q gemeinsam höchstens 2 ist, daß, wenn Y ein Schwefel- oder Sauerstoffatom oder ein Sulfinylradikal bedeutet, P für eine gegebenenfalls substituierte Alkylenkette steht und daß, wenn Y ein Sauerstoffatom oder ein Sulfinylradikal bedeutet, Q für eine gegebenenfalls substituierte Alkylenkette mit 2 bis 4 Kohlenstoffatomen steht;

$R^1$ für ein Wasserstoffatom oder ein Alkylradikal mit 1 bis 10 Kohlenstoffatomen steht;

A für ein 3,4-Dioxocyclobuten-1,2-diyl-Radikal oder ein Radikal der Formel C=Z steht, wobei Z ein Schwefel- oder Sauerstoffatom oder ein Radikal der Formel NCN, $NNO_2$, $CHNO_2$, $NCONH_2$, $C(CN)_2$, $NCOR^2$, $NCO_2R^2$, $NSO_2R^2$ oder $NR^3$ bedeutet, wobei $R^2$ ein Alkylradikal mit 1 bis 6 Kohlenstoffatomen oder ein Arylradikal mit 6 bis 12 Kohlenstoffatomen und $R^3$ ein Wasserstoffatom oder ein Alkylradikal mit 1 bis 6 Kohlenstoffatomen ist;

B für ein Alkoxy oder Alkylthioradikal mit 1 bis 6 Kohlenstoffatomen oder ein Radikal der Formel $NR^4R^5$ steht, wobei $R^4$ und $R^5$, welche gleich oder verschieden sein können, Wasserstoffatome, Alkylradikale mit 1 bis 10 Kohlenstoffatomen, Alkenyl- oder Alkinylradikale mit 3 bis 6 Kohlenstoffatomen, deren Doppel- bzw. Dreifachbindung vom Stickstoffatom von $NR^4R^5$ durch mindestens ein Kohlenstoffatom getrennt ist, (primäre Hydroxy)alkyl- oder (primäre Amino)alkylradikale mit 2 bis 6 Kohlenstoffatomen oder Cycloalkylradikale mit 3 bis 6 Kohlenstoffatomen bedeuten oder $R^4$ und $R^5$ miteinander verbunden sind, so daß gemeinsam mit dem Stickstoffatom, an das sie geknüpft sind, ein 5- oder 6-gliedriger gesättigter Ring gebildet wird, der gegebenenfalls zusätzlich ein Sauerstoffatom oder NH -Radikal enthält;

sowie die pharmazeutisch zulässigen Säureadditionssalze davon.

2. Heterocyclisches Derivat nach Anspruch 1, worin $R^1$ für ein Wasserstoffatom oder ein Methyl- oder n-Butylradikal steht; P gegebenenfalls durch 1 oder 2 Methylradikale substituiert ist; Q gegebenenfalls durch 1 oder 2 Methylradikale substituiert ist; $R^2$ für ein Methyl-, Phenyl- oder p-Tolylradikal steht; $R^3$ für ein Wasserstoffatom oder ein Methylradikal steht; $R^3$ für ein Wasserstoffatom oder ein Methylradikal steht; B für ein Methoxy-, Äthoxy- oder Methylthioradikal oder ein Radikal der Formel $NR^4R^5$ steht, wobei $R^4$ und $R^5$, welche gleich oder verschieden sein können, Wasserstoffatome oder Methyl-, Äthyl-, n-Propyl-, i-Propyl-, n-Hexyl-, Allyl-, Propargyl-, 2-Hydroxyäthyl-, 3-Hydroxy -propyl-, 2-Aminoäthyl-, Cyclopropyl-, Cyclobutyl- oder Cyclohexylradikale bedeuten oder $R^4$ und $R^5$ miteinander verbunden sind, so daß sie gemeinsam mit dem Stickstoffatom, an das sie geknüpft sind, einen Pyrrolidin-, Piperidin-, Piperazin- oder Morpholinring bilden.

3. Heterocyclisches Derivat nach Anspruch 1 oder 2, worin Y für Schwefelatom steht, P für ein

gegebenenfalls substituiertes Methylenradikal steht und Q für ein gegebenenfalls substituiertes Äthylenradikal steht.

4. Heterocyclisches Derivat nach Anspruch 1 oder 2, worin Y für eine direkte Bindung steht, P für ein gegebenenfalls substituiertes Methylenradikal steht und Q für ein gegebenenfalls substituiertes Propylen- oder Butylenradikal steht.

5. Heterocyclisches Derivat nach Anspruch 3 oder 4, worin die Anzahl der Alkylradikalsubstituenten an P und Q gemeinsam 1 ist.

6. Heterocyclisches Derivat nach einem der Ansprüche 1 bis 5, worin $R^1$ für ein Wasserstoffatom steht und B für ein Radikal der Formel $NR^4R^5$ steht, wobei $R^5$ ein Wasserstoffatom und $R^4$ ein Wasserstoffatom oder ein Methyl-, Äthyl-, n-Propyl-, i-Propyl-, n-Hexyl-, Allyl-, Propargyl-, 2-Hydroxyäthyl-, 3-Hydroxypropyl-, 2-Aminoäthyl-, Cyclopropyl-, Cyclobutyl- oder Cyclohexylradikal bedeutet.

7. Heterocyclisches Derivat nach einem der Ansprüche 1 bis 6, worin D für ein Schwefelatom steht und E für ein CH -Radikal oder ein Stickstoffatom steht.

8. 2-Guanidino-4-[5-(2-cyano-3-methylguanidino)-2-methylpentyl]thiazol, 2-Guanidino-4[2-(2-cyano-3-methylguanidino)-1-methyläthylthiomethyl]thiazol und 2-Guanidino-4-[2-(2-cyano-3-methylguanidino)-2-methyläthylthiomethyl]thiazol sowie die pharmazeutisch zulässigen Säureadditionssalze davon.

9. Verfahren zur Herstellung eines heterocyclischen Derivats nach Anspruch 1, gekennzeichnet durch

(a) Umsetzung einer Verbindung der Formel II:

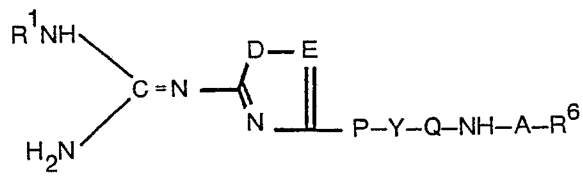

II

worin $R^6$ für ein ersetzbares Radikal steht, mit einer Verbindung der Formel B—H;

(b) für diejenigen Verbindungen, worin A für ein Radikal der Formel C=Z steht, wobei Z ein Schwefel- oder Sauerstoffatom bedeutet, und B für ein Radikal der Formel $NR^4R^5$ steht, wobei $R^5$ ein Wasserstoffatom bedeutet und $R^4$ mit Ausnahme eines Hydroxyalkyl- oder Aminoalkylradikals die in Anspruch 1 angegebene Bedeutung besitzt, Umsetzung einer Verbindung der Formel III:

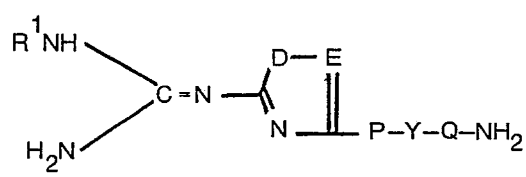

III

mit einer Verbindung der Formel $R^7$—N=C=G, worin G für ein Sauerstoff- oder Schwefelatom steht und $R^7$ für ein Wasserstoffatom oder ein Alkyl-, Alkenyl-, Alkinyl- oder Cycloalkylradikal steht;

(c) Umsetzung einer Verbindung der Formel III mit einer Verbindung der Formel IV:

$$R^6—A—B \qquad IV$$

worin $R^6$ für ein ersetzbares Radikal steht;

(d) für diejenigen Verbindungen, worin A für ein Radikal der Formel C=Z steht, wobei Z ein Radikal der Formel $NCONH_2$ bedeutet, und B für ein Radikal der Formel $NR^4R^5$ steht, Hydrolyse einer Verbindung der Formel I, worin A für ein Radikal der Formel C=Z steht, wobei Z ein Radikal der Formel NCN bedeutet, und B für ein Radikal der Formel $NR^4R^5$ steht;

(e) für diejenigen Verbindungen, worin Y für ein Sulfinylradikal steht, Oxidation einer Verbindung der Formel I, worin Y für ein Schwefelatom steht; oder

(f) für eine Verbindung, die ein optisch aktives Enantiomer ist, Trennung der racemischen Verbindung der Formel I oder Anwendung eines der Verfahren (a) bis (d), wobei das Ausgangsmaterial selbst ein getrenntes Isomer ist;

worauf, wenn die Verbindung der Formel I in Form der freien Base anfällt und ein Salz gewünscht wird, die freie Base mit einer Säure umgesetzt wird, die ein pharmazeutisch zulässiges Anion liefert.

10. Pharmazeutische Zusammensetzung, welche ein heterocyclisches Derivat nach Anspruch 1 gemeinsam mit einem nichtgiftigen pharmazeutisch zulässigen Verdünnungsmittel oder Trägermittel enthält.

11. Heterocyclisches Derivat der Formel:

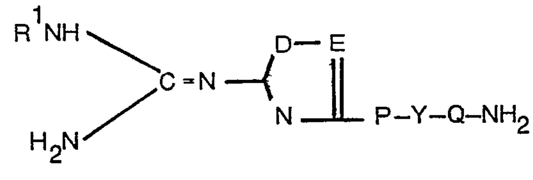

III

worin
D für ein Sauerstoff- oder Schwefelatom oder ein NH -Radikal steht;
E für ein CH -Radikal oder, wenn D ein Sauterstoff- oder Schwefelatom bedeutet, ein Stickstoffatom steht;
Y für ein Schwefel- oder Sauerstoffatom, eine direkte Bindung oder ein Methylen- oder Sulfinylradikal steht;
P für eine direkte Bindung oder eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, und Q für eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, mit der Maßgabe, daß mindestens eines der Symbole P und Q durch mindestens ein Alkylradikal substituiert ist, daß die Anzahl der Alkylradikalsubstituenten an P und Q gemeinsam höchstens 2 ist, daß, wenn Y ein Schwefel- oder Sauerstoffatom oder ein Sulfinylradikal bedeutet, P für eine gegebenenfalls substituierte Alkylenkette steht und daß, wenn Y ein Sauerstoffatom oder ein Sulfinylradikal bedeutet, Q für eine gegebenenfalls substituierte Alkylenkette mit 2 bis 4 Kohlenstoffatomen steht; und
$R^1$ für ein Wasserstoffatom oder ein Alkylradikal mit 1 bis 10 Kohlenstoffatomen steht;
sowie die Säureadditionssalze davon.

**Patentanspruch für den Vertragsstaat: AT**

Verfahren zur Herstellung eines heterocyclischen Derivats der Formel

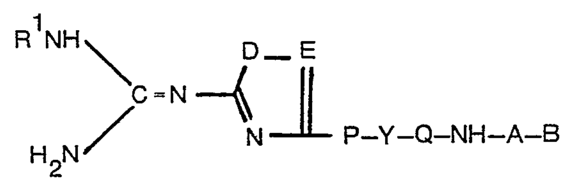

worin
D für ein Sauerstoff- oder Schwefelatom oder ein NH -Radikal steht;
E für ein CH -Radikal oder, wenn D ein Sauerstoff- oder Schwefelatom bedeutet, ein Stickstoffatom steht;
Y für ein Schwefel- oder Sauerstoffatom, eine direkte Bindung oder ein Methylen- oder Sulfinylradikal steht;
P für eine direkte Bindung oder eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, und Q für eine unverzweigte Alkylenkette mit 1 bis 4 Kohlenstoffatomen steht, die gegebenenfalls durch 1 oder 2 Alkylradikale mit 1 bis 4 Kohlenstoffatomen substituiert ist, mit der maßgabe, daß mindestens eines der Symbole P und Q durch mindestens ein Alkylradikal substituiert ist, daß die Anzahl der Alkylradikalsubstituenten an P und Q gemeinsam höchstens 2 ist, daß, wenn Y ein Schwefel- oder Sauerstoffatom oder ein Sulfinylradikal bedeutet, P für eine gegebenenfalls substituierte Alkylenkette steht und daß, wenn Y ein Sauerstoffatom oder ein Sulfinylradikal bedeutet, Q für eine gegebenenfalls substituierte Alkylenkette mit 2 bis 4 Kohlenstoffatomen steht;
$R^1$ für ein Wasserstoffatom oder ein Alkylradikal mit 1 bis 10 Kohlenstoffatomen steht;
A für ein 3,4-Dioxocyclobuten-1,2-diyl-Radikal oder ein Radikal der Formel C=Z steht, wobei Z ein Schwefel- oder Sauerstoffatom oder ein Radikal der Formel NCN, $NNO_2$, $CHNO_2$, $NCONH_2$, $C(CN)_2$, $NCOR^2$, $NCO_2R^2$, $NSO_2R^2$ oder $NR^3$ bedeutet, wobei $R^2$ ein Alkylradikal mit 1 bis 6 Kohlenstoffatomen oder ein Arylradikal mit 6 bis 12 Kohlenstoffatomen und $R^3$ ein Wasserstoffatom oder ein Alkylradikal mit 1 bis 6 Kohlenstoffatomen ist;
B für ein Alkoxy oder Alkylthioradikal mit 1 bis 6 Kohlenstoffatomen oder ein Radikal der Formel $NR^4R^5$ steht, wobei $R^4$ und $R^5$, welche gleich oder verschieden sein können, Wasserstoffatome, Alkylradikale mit 1 bis 10 Kohlenstoffatomen, Alkenyl- oder Alkinylradikale mit 3 bis 6 Kohlenstoffatomen, deren Doppel- bzw. Dreifachbindung vom Stickstoffatom von $NR^4R^5$ durch mindestens ein Kohlenstoffatom getrennt ist, (primäre Hydroxy)alkyl- oder (primäre Amino)alkylradikale mit 2 bis 6 Kohlenstoffatomen oder Cycloalkylradikale mit 3 bis 6 Kohlenstoffatomen bedeuten oder $R^4$ und $R^5$ miteinander verbunden sind, so daß gemeinsam mit dem Stickstoffatom, an das sie geknüpft sind, ein 5- oder 6-

gliedriger gesättigter Ring gebildet wird, der gegebenenfalls zusätzlich ein Sauerstoffatom oder NH - Radikal enthält;

sowie der pharmazeutisch zulässigen Säureadditionssalze davon, gekennzeichnet durch

(a) Umsetzung einer Verbindung der Formel:

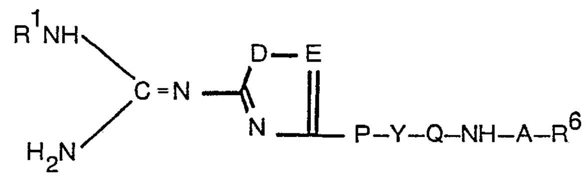

II

worin $R^6$ für ein ersetzbares Radikal steht, mit einer Verbindung der Formel B—H;

(b) für diejenigen Verbindungen, worin A für ein Radikal der Formel C=Z steht, wobei Z ein Schwefel- oder Sauerstoffatom bedeutet, und B für ein Radikal der Formel $NR^4R^5$ steht, wobei $R^5$ ein Wasserstoffatom bedeutet und $R^4$ mit Ausnahme eines Hydroxyalkyl- oder Aminoalkylradikals die in Anspruch 1 angegebene Bedeutung besitzt, Umsetzung einer Verbindung der Formel:

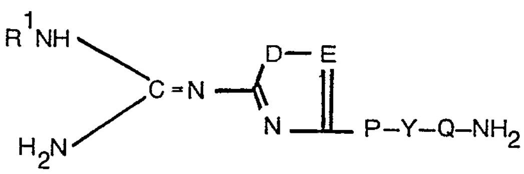

III

mit einer Verbindung der Formel $R^7$—N=C=G, worin G für ein Sauerstoff- oder Schwefelatom steht und $R^7$ für ein Wasserstoffatom oder ein Alkyl-, Alkenyl-, Alkinyl- oder Cycloalkylradikal steht;

(c) Umsetzung einer Verbindung der Formel III mit einer Verbindung der Formel:

$$R^6—A—B \qquad IV$$

worin $R^6$ für ein ersetzbares Radikal steht;

(d) für diejenigen Verbindungen, worin A für ein Radikal der Formel C=Z steht, wobei Z ein Radikal der Formel $NCONH_2$ bedeutet, und B für ein Radikal der Formel $NR^4R^5$ steht, Hydrolyse einer Verbindung der Formel I, worin A für ein Radikal der Formel C=Z steht, wobei Z ein Radikal der Formel NCN bedeutet, und B für ein Radikal der Formel $NR^4R^5$ steht;

(e) für diejenigen Verbindungen, worin Y für ein Sulfinylradikal steht, Oxidation einer Verbindung der Formel I, worin Y für ein Schwefelatom steht; oder

(f) für eine Verbindung, die ein optisch aktives Enantiomer ist, Trennung der racemischen Verbindung der Formel I oder Anwendung eines der Verfahren (a) bis (d), wobei das Ausgangsmaterial selbst ein getrenntes Isomer ist;

worauf, wenn die Verbindung der Formel I in Form der freien Base anfällt und ein Salz gewünscht wird, die freie Base mit einer Säure umgesetzt wird, die ein pharmazeutisch zulässiges Anion liefert.

**Revendications pour les Etats contractants: BE CH DE FR GB IT NL SE**

1. Dérivé hétérocyclique de formule:

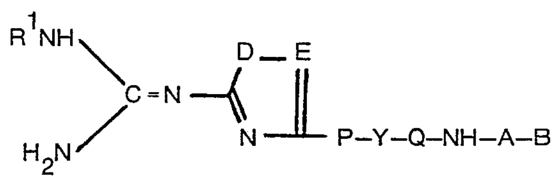

I

dans laquelle

D est un atome d'oxygène ou de soufre ou un radical NH;

E est un radical CH ou, lorsque D est un atome d'oxygène ou de soufre, E est un atome d'azote;

Y est un atome de soufre ou d'oxygène, un liaison simple ou un radical méthylène ou sulfinyle;

P est une liaison simple ou une chaîne alkylène non ramifiée de 1 à 4 atomes de carbone éventuellement substituée par 1 ou 2 radicaux alkyle ayant 1 à 4 atomes de carbone, et Q est une chaîne alkylène non ramifiée ayant 1 à 4 atomes de carbone, éventuellement substituée par 1 ou 2 radicaux alkyle ayant 1 à 4 atomes de carbone, sous réserve qu'au moins l'un de P et Q soit substitué par au

moins 1 radical alkyle, que le nombre de substituants alkyle sur P et Q considérés ensemble soit au maximum égal à 2, que lorsque Y est un atome de soufre ou d'oxygène ou un radical sulfinyle, P soit une chaîne alkylène éventuellement substituée et que lorsque Y est un atome d'oxygène ou un radical sulfinyle, Q soit une chaîne alkylène éventuellement substituée ayant 2 à 4 atomes de carbone;
$R^1$ est un atome d'hydrogène ou un radical alkyle ayant 1 à 10 atomes de carbone;
A est un radical 3,4-dioxocyclobutène-1,2-diyle ou un radical de formule C=Z, dans laquelle Z est un atome de soufre ou d'oxygène ou un radical de formule NCN, $NNO_2$, $CHNO_2$, $NCONH_2$, $C(CN)_2$, $NCOR^2$, $NCO_2R^2$, $NSO_2R^2$ ou $NR^3$ où $R^2$ est un radical alkyle ayant 1 à 6 atomes de carbone ou un radical aryle ayant 6 à 12 atomes de carbone et $R^3$ est un atome d'hydrogène ou un radical alkyle ayant 1 à 6 atomes de carbone;
B est un radical alkoxy ou alkylthio ayant 1 à 6 atomes de carbone ou un radical de formule $NR^4R^5$ dans laquelle $R^4$ et $R^5$, qui peuvent être égaux ou différents, représentent des atomes d'hydrogène, des radicaux alkyle ayant 1 à 10 atomes de carbone, des radicaux alcényle ou alcynyle ayant 3 à 6 atomes de carbone dont la liaison double ou la liaison triple, respectivement, est séparée de l'atome d'azote de $NR^4R^5$ par au moins un atome de carbone, des radicaux (hydroxy primaire)-alkyle ou (amino primaire)-alkyle ayant 2 à 6 atomes de carbone ou des radicaux cycloalkyle ayant 3 à 6 atomes de carbone, ou bien $R^4$ et $R^5$ forment conjointement avec l'atome d'azote auquel ils sont liés un noyau pentagonal ou hexagonal saturé qui renferme éventuellement un autre atome d'oxygène ou radical NH;
et les sels d'addition d'acides pharmaceutiquement acceptables de ce dérivé.

2. Dérivé hétérocyclique suivant la revendication 1, dans lequel $R^1$ est un atome d'hydrogène ou un radical méthyle ou n-butyle; P est éventuellement substitué par 1 ou 2 radicaux méthyle; Q est éventuellement substitué par 1 ou 2 radicaux méthyle; $R^2$ est un radical méthyle, phényle ou p-tolyle; $R^3$ est un atome d'hydrogène ou un radical méthyle; B est un radical méthoxy, éthoxy ou méthylthio ou un radical de formule $NR^4R^5$ dans laquelle $R^4$ et $R^5$, qui peuvent être égaux ou différents, sont des atomes d'hydrogène ou des radicaux méthyle, éthyle, n-propyle, isopropyle, n-hexyle, allyle, propargyle, 2-hydroxyéthyle, 3-hydroxypropyle, 2-amino-éthyle, cyclopropyle, cyclobutyle ou cyclohexyle, ou bien $R^4$ et $R^5$ forment conjointement avec l'atome d'azote auquel ils sont liés un noyau de pyrrolidine, de pipéridine, de pipérazine ou de morpholine.

3. Dérivé hétérocyclique suivant la revendication 1 ou 2, dans lequel Y est un atome de soufre, P est un radical méthylène éventuellement substitué et Q est un radical éthylène éventuellement substitué.

4. Dérivé hétérocyclique suivant la revendication 1 ou 2, dans lequel Y est une liaison simple, P est un radical méthylène éventuellement substitué et Q est un radical propylène ou butylène éventuellement substitué.

5. Dérivé hétérocyclique suivant la revendication 3 ou 4, dans lequel le nombre de radicaux alkyle portés comme substituants par P et Q ensemble est égal à 1.

6. Dérivé hétérocyclique suivant l'une quelconque des revendications 1 à 5, dans lequel $R^1$ est un atome d'hydrogène et B est un radical de formule $NR^4R^5$ dans laquelle $R^5$ est un atome d'hydrogène et $R^4$ est un atome d'hydrogène ou un radical méthyle, éthyle, n-propyle, isopropyle, n-hexyle, allyle, propargyle, 2-hydroxyéthyle, 3-hydroxypropyle, 2-amino-éthyle, cyclopropyle, cyclobutyle ou cyclohexyle.

7. Dérivé hétérocyclique suivant l'une quelconque des revendications 1 à 6, dans lequel D est un atome de soufre et E est un radical CH ou un atome d'azote.

8. Le 2-guanidino-4-[5-(2-cyano-3-méthylеguanidino)-2-méthylpentyl]thiazole, le 2-guanidino-4-[2-(2-cyano-3-méthylguanidino)-1-méthyléthylthiométhyl]thiazole et le 2-guanidino-4-[2-(2-cyano-3-méthylguanidino)-2-méthyléthylthiométhyl]thiazole et leurs sels d'addition d'acides pharmaceutiquement acceptables.

9. Procédé de production d'un dérivé hétérocyclique suivant la revendication 1, caractérisé par:
(a) la réaction d'un composé de formule II:

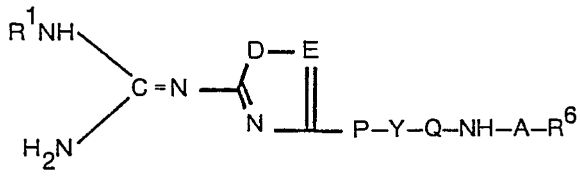

II

dans laquelle $R^6$ est un radical déplaçable, acec un composé de formule B—H;

(b) pour les composés dans lesquels A est un radical de formule C—Z dans laquelle Z est un atome de soufre ou d'oxygène et B est un radical de formule $NR^4R^5$ dans laquelle $R^5$ est un atome d'hydrogène et $R^4$ a la valeur indiquée dans la revendication 1 autre qu'un radical hydroxyalkyle ou amino-alkyle, la réaction d'un composé de formula III:

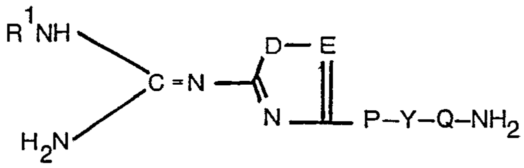

III

avec un composé de formule $R^7$—N=C=G dans laquelle G est un atome d'oxygène ou de soufre et $R^7$ est un atome d'hydrogène ou un radical alkyle, alcényle, alcynyle ou cycloalkyle;

(c) la réaction d'un composé de formule III avec un composé de formule IV:

$$R^6—A—B \qquad IV$$

dans laquelle $R^6$ est un radical déplaçable;

(d) pour les composés dans lesquels A est un radical de formule C=Z dans laquelle Z est un radical de formule $NCONH_2$ et B est un radical de formule $NR^4R^5$, l'hydrolyse d'un composé de formule I dans laquelle A est un radical de formule C=Z dans laquelle Z est un radical de formule NCN et B est un radical de formule $NR^4R^5$;

(e) pour les composés dans lesquels Y est un radical sulfinyle, l'oxydation d'un composé de formule I dans laquelle Y est un atome de soufre; ou

(f) pour un composé qui est un énantiomère optiquement actif, la résolution du composé racémique de formule I ou l'utilisation de l'un quelconque des procédés (a) à (d) inclus dans lequel la matière de départ est elle-même un isomère dédoublé;

après quoi, lorsque le composé de formule I est obtenu sous la forme de la base libre et que l'on désire un sel, on fait réagir la base libre avec un acide qui apporte un anion pharmaceutiquement acceptable.

10. Composition pharmaceutique comprenant un dérivé hétérocyclique suivant la revendication 1, en association avec un diluant ou support non toxique pharmaceutiquement acceptable.

11. Dérivé hétérocyclique de formule:

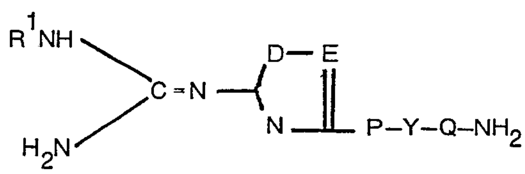

III

dans laquelle
D est un atome d'oxygène ou de soufre ou un radical NH;
E est un radical CH ou bien, lorsque D est un atome d'oxygène ou de soufre, E est un atome d'azote;
Y est un atome de soufre ou d'oxygène, une liaison simple ou un radical méthylène ou sulfinyle;
P est une liaison simple ou une chaîne alkylène non ramifié de 1 à 4 atomes de carbone, éventuellement substituée par 1 ou 2 radicaux alkyle ayant 1 à 4 atomes de carbone, et Q est une chaîne alkylène non ramifiée ayant 1 à 4 atomes de carbone, éventuellement substituée par 1 ou 2 radicaux alkyle ayant 1 à 4 atomes de carbone, sous réserve qu'au moins l'un de P et Q soit substitué par au moins 1 radical alkyle, que le nombre de radicaux alkyle portés comme substituants par P et Q conjointement soit au maximum égal à 2, que lorsque Y est un atome de soufre ou d'oxygène ou un radical sulfinyle, P soit une chaîne alkylène éventuellement substituée et que lorsque Y est un atome d'oxygène ou un radical sulfinyle, Q soit une chaîne alkylène de 2 à 4 atomes de carbone éventuellement substituée; et
$R^1$ est un atome d'hydrogène ou un radical alkyle ayant 1 à 10 atomes de carbone;
et ses sels d'addition d'acides.

**Revendication pour l'Etat contractant: AT**

Procédé de production d'un dérivé hétérocyclique de formule:

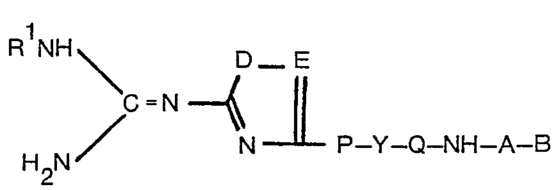

I

dans laquelle

D est un atome d'oxygène ou de soufre ou un radical NH; E est un radical CH ou, lorsque D est un atome d'oxygène ou de soufre, E est un atome d'azote; Y est un atome de soufre ou d'oxygène, une liaison simple ou un radical méthylène ou sulfinyle; P est une liaison simple ou une chaîne alkylène non ramifiée de 1 à 4 atomes de carbone éventuellement substituée par 1 ou 2 radicaux alkyle ayant 1 à 4 atomes de carbone, et Q est une chaîne alkylène non ramifiée ayant 1 à 4 atomes de carbone, éventuellement substituée par 1 ou 2 radicaux alkyle ayant 1 à 4 atomes de carbone, sous réserve qu'au moins l'un de P et Q soit substitué par au moins 1 radical alkyle, que le nombre de substituants alkyle sur P et Q considérés ensemble soit au maximum égal à 2, que lorsque Y est un atome de soufre ou d'oxygène ou un radical sulfinyle, P soit une chaîne alkylène éventuellement substituée et que lorsque Y est un atome d'oxygène ou un radical sulfinyle, Q soit une chaîne alkylène éventuellement substituée ayant 2 à 4 atomes de carbone; $R^1$ est un atome d'hydrogène ou un radical alkyle ayant 1 à 10 atomes de carbone; A est un radical 3,4-dioxocyclobutène-1,2-diyle ou un radical de formule C=Z, dans laquelle Z est un atome de soufre ou d'oxygène ou un radical de formule NCN, $NNO_2$, $CHNO_2$, $NCONH_2$, $C(CN)_2$, $NCOR^2$, $NCO_2R^2$, $NSO_2R^2$ ou $NR^3$ où $R^2$ est un radical alkyle ayant 1 à 6 atomes de carbone ou un radical aryle ayant 6 à 12 atomes de carbone et $R^3$ est un atome d'hydrogène ou un radical alkyle ayant 1 à 6 atomes de carbone;
B est un radical alkoxy ou alkylthio ayant 1 à 6 atomes de carbone ou un radical de formule $NR^4R^5$ dans laquelle $R^4$ et $R^5$, qui peuvent être égaux ou différents, représentent des atomes d'hydrogène, des radicaux alkyle ayant 1 à 10 atomes de carbone, des radicaux alcényle ou alcynyle ayant 3 à 6 atomes de carbone dont la liaison double ou la liaison triple, respectivement, est séparée de l'atome d'azote de $NR^4R^5$ par un moins un atome de carbone, des radicaux (hydroxy primaire)-alkyle ou (amino primaire)-alkyle ayant 2 à 6 atomes de carbone ou des radicaux cycloalkyle ayant 3 à 6 atomes de carbone, ou bien $R^4$ et $R^5$ forment conjointement avec l'atome d'azote auquel ils sont liés un noyau pentagonal ou hexagonal saturé qui renferme éventuellement un autre atome d'oxygène ou radical NH;
et de ses sels d'addition d'acides pharmaceutiquement acceptables; caractérisé par:

(a) la réaction d'un composé de formule:

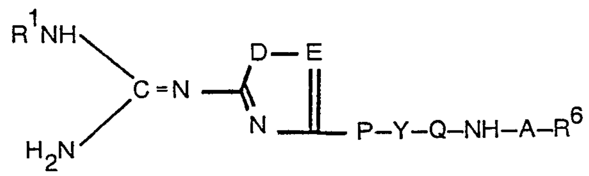

II

dans laquelle $R^6$ est un radical déplaçable, avec un composé de formule B—H;

(b) pour les composés dans lesquels A est un radical de formule C=Z dans laquelle Z est un atome de soufre ou d'oxygène et B est un radical de formule $NR^4R^5$ dans laquelle $R^5$ est un atome d'hydrogène et $R^4$ a la valeur indiquée précédemment autre qu'un radical hydroxyalkyle ou amino-alkyle, la réaction d'un composé de formule:

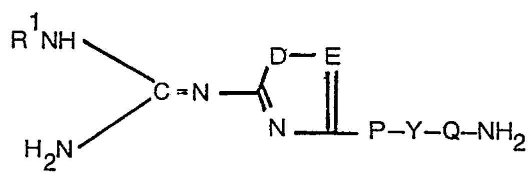

III

avec un composé de formule $R^7$—N=C=G dans laquelle G est un atome d'oxygène ou de soufre et $R^7$ est un atome d'hydrogène ou un radical alkyle, alcényle, alcynyle ou cycloalkyle;

(c) la réaction d'un composé de formule III avec un composé de formule:

$$R^6—A—B \qquad IV$$

dans laquelle $R^6$ est un radical déplaçable;

(d) pour les composés dans lesquels A est un radical de formule C=Z dans laquelle Z est un radical de formule $NCONH_2$ et B est un radical de formule $NR^4R^5$, l'hydrolyse d'un composé de formule I dans laquelle A est un radical de formule C=Z dans laquelle Z est un radical de formule NCN et B est un radical de formule $NR^4R^5$;

(e) pour les composés dans lesquels Y est un radical sulfinyle, l'oxydation d'un composé de formule I dans laquelle Y est un atome de soufre; ou

(f) pour un composé qui est un énantiomère optiquement actif, la résolution du composé racémique de formule I ou l'utilisation de l'un quelconque des procédés (a) à (d) inclus dans lequel la matière de départ est elle-même un isomère dédoublé;

après quoi, lorsque le composé de formule I est obtenu sous la forme de la base libre et que l'on désire un sel, on fait réagir la base libre avec un acide qui apporte un anion pharmaceutiquement acceptable.